# EUROPEAN PATENT APPLICATION

(11) **EP 4 700 709 A1**
(43) Date of publication of application: **25.02.2026**
(21) Application number: 24306382.3
(22) Date of filing: 21.08.2024
(51) Int. Cl.: G06T 15/08, G06T 7/11, G16H 30/00

(54) **SELECTIVE VOLUME RENDERING**

(71) Applicant: Koninklijke Philips N.V., 5656 AG Eindhoven (NL)
(72) Inventor: WIEMKER, Rafael, 5656 Eindhoven (NL); ROUET, Jean-Michel, 5656 Eindhoven (NL); VILLIEN, Marjorie, 5656 Eindhoven (NL); VLACHOMITROU, Anna Sesilia, 5656 Eindhoven (NL); MATUTE FLORES, Jose Alejandro, 5656 Eindhoven (NL); HEESE, Harald, 5656 Eindhoven (NL); NICKISCH, Hannes, 5656 Eindhoven (NL); PETERS, Jochen, 5656 Eindhoven (NL)
(74) Representative: Philips Intellectual Property & Standards

(57) **Abstract**

The present disclosure concerns a computer-implemented method for volume rendering of a region comprising a structure in a volume of interest. The method comprises receiving data representing a plurality of voxels of the volume of interest representative of the region. The method comprises obtaining a starting point voxel representative of a starting point anatomy which is connected to the structure. The method comprises determining an alignment value indicative of a direction alignment of the respective voxel relative to neighbored voxels in a neighborhood of the respective voxel. The method comprises determining, based on the alignment value per voxel, a connectivity value per voxel of the plurality of voxels relative to the starting point voxel, wherein the connectivity value is indicative of an anatomical connection relative to the starting point anatomy. The method comprises identifying, based on the connectivity value, voxels of interest representing connected structures in the region comprising the structure. The method comprises volume rendering of the voxels of interest for visualizing the structure.

## Description

### FIELD OF THE INVENTION

The present disclosure is directed to a computer-implemented method of volume rendering, a data processing apparatus comprising means for carrying out said method, a computer program and a computer-readable medium.

### BACKGROUND OF THE INVENTION

Spatial resolution in medical images obtained by medical imaging devices, such as CT and in particular Photon Counting CT scanners, is increased and may allow for more detailed anatomical and/or pseudo-functional visualization for diagnostic appraisal. Direct volume rendering (DVR) may visualize in particular delicate and complex details without requiring an underlying explicit segmentation.

A downside of a higher spatial resolution in 3D volumes of interest may be that comprehensive, undiscriminating (direct) volume rendering may clutter and/or occlude the visualization with - in terms of diagnosis - less relevant other anatomical structures.

It is desired to improve volume rendering such that an anatomy of interest is more clearly visualized so as to help to improve diagnosis.

### SUMMARY OF THE INVENTION

The present invention solves this problem and provides a computer-implemented method for volume rendering of at least one region comprising at least part of a structure in a volume of interest according to claim 1.

In other words, the volume of interest is reflective of a region of interest that comprises part of a structure of interest.
In particular, the step of volume rendering may be a form of direct volume rendering. In another example, the structure may be an anatomical structure or at least a part thereof, such as a part of a vascular structure.

The method comprises the step of receiving data representing a plurality of voxels of the volume of interest representative of the at least one region. The at least one region may for example be a region of interest comprising at least part of an anatomical structure of interest in the volume of interest. For example, the region of interest is an anatomical region of interest such as a vasculature region.

The method comprises the step of obtaining, in the volume of interest, at least one starting point voxel representative of a starting point anatomy which is connected to the at least part of a structure. For example, the starting point anatomy is connected to the structure and the starting point voxel is a voxel that forms and/or coincides with the starting point anatomy. In particular, the structure is an anatomical structure, and the starting point anatomy is an anatomy that is connected to and/or forms part of that anatomical structure. For example, the starting point anatomy is the aorta, and the structure is the coronary system. The starting point voxel may then be a voxel that represents part of the aorta.

The method comprises the step of determining, per voxel of the plurality of voxels of the volume of interest, an alignment value indicative of a direction (D) alignment of the respective voxel relative to neighbored voxels in a neighborhood (N) of the respective voxel.

The neighborhood is an area located around the respective voxels where and can be defined using a criterion like a predefined Euclid distance and/or a threshold for a neighborhood distance. A neighborhood voxel may also be immediate neighbors, i.e. voxels that share a common surface and/or corner with the voxel of interest. In contrast, indirect neighbors would be voxels that lie within the neighborhood but exclude the immediate neighbors.

The alignment value represents the direction alignment of the respective voxel to the neighbored voxels, i.e. the voxels that lie within the neighborhood. The alignment value may be a scalar alignment value.

The method comprises the step of, based on the alignment value per voxel, determining a connectivity value per voxel of the plurality of voxels relative to the starting point voxel, the connectivity value being indicative of an anatomical connection relative to the starting point anatomy.

The anatomical connection may for example be an anatomical vascular connection.

The method comprises the step of, based on the connectivity value, identifying voxels of interest representing connected structures in the at least one region comprising the at least one structure.

The method comprises the step of volume rendering of at least the voxels of interest for visualizing the at least part of a structure.

The present invention may be seen as selective volume rendering using directional connectivity for diagnostic visualization and navigation. It may help in navigating to locations and/or areas of diagnostic interest and/or helps in making a diagnosis. Selective means that instead of rendering the whole volume only a selective part of the volume is rendered. For example, scalar-based rendering per voxel may be performed based on alignment of vectors of voxels in the neighborhood.

In particular, the present invention may also be summarized as volume rendering of a structure of interest by using direction information of voxels and alignment of such direction information between voxels, wherein the direction information is obtained based on, i.e. derived from, 3D image data.

In a preferred embodiment, the invention may be seen as combining an automatic detection of a robustly detectable key component (e.g. of the vasculature, such as the ascending aorta) as starting point anatomy, with a propagation of connectedness e.g. by virtue of the local Hessian matrix (reflective of second order-derivatives) and structure tensor eigenvectors.

The method, in its entirety or partly, i.e. only some steps thereof, may be carried out by a computer.

The advantage of selective volume rendering and/or rendering at least the voxel of interest for visualizing at least part of a structure is that it allows for rendering of anatomies of interest. It may overcome the problem of direct volume rendering where all image voxels of a volume interest are rendered that typically yields an overcrowded rendering which multiple mutually occluding anatomical structures. In other words, the invention provides for rendering of selected anatomies of relevance, as desired.

Preferably, the steps of the invention are automated, which allows for a more time-efficient rendering in contrast to manual selection of delicate structures within the volume of interest which are known to be tedious and time-costly effort. In other words, the method of an embodiment of the invention may be more time-efficient.

Another advantage of the invention is that no machine learning based segmentation algorithm is required to provide a visualization of structures of interest. In contrast, the selective visualization of structures of interest is obtained through rendering according to the invention. Typically, machine learning based automatic segmentation algorithms require a wide as well as representative training data base with costly careful curation and annotation and might be prone to error for unusual variants or diseases, or non-anatomical artifacts such as stents and bypasses.

The invention may provide for automatically rendering of only salient yet delicate and variable vascular structures, in particular the coronary arteries, without a need for discrete segmentation which would require massive training and/or might be prone to error for unusual anatomical variants.

Another advantage of selective volume rendering according to the invention is that it allows for fully automatically rendering of structures of interest without requiring manual clipping of unwanted anatomies (that are not of interest). The invention may still allow for adjusting visualization settings interactively afterwards.

Another advantage of selective volume rendering is that it may allow for visualizing delicate and intertwined structures (e.g., arteries vs. veins) of a priori unknown shape as well as presence and number of occurrences. The voxels being rendered depends on to what extend the voxel (of a potential artery or vein) is connected to a well known starting point anatomy such as an ascending aorta. In other words, what is being rendered depends on the propagation of connectivity with respect to a robustly detectable key component. This is a surprisingly efficient approach to visualize delicate and intertwined structures.

The invention may also allow visual diagnostic appraisal, and/or quick navigation to locations of interest in a standard slice-wise-viewer (e.g. mouse click on volume rendering bringing up the corresponding image slice with a marker).

Another advantage of the invention compared to `black-box' machine learning (ML-) models is that the invention relies on a mathematical and intuitive concept which does not require a training data base with is rare and costly and may require careful curation and annotation efforts.

The present invention may be used for volume rendering of a volume of interest based on 3D image data that has been reconstructed based on raw data acquired by a medical imaging modality. For example, such a medical imaging modality may be one of CT imaging, MR imaging, SPECT imaging, PET imaging and/or ultrasound imaging. The invention is therefore not limited to volumes of interest and/or 3D image data obtained via CT imaging.

The features of volume of interest and 3D image data may be used interchangeably. In particular, a volume of interest is a subset of the 3D image data, namely the volume (subset) in the 3D image data that is of interest, because it at least partly comprises a region and/or structure of interest.

The invention allows for improved volume rendering of 3D image data of the anatomical structures, in particular of the vasculature. Specifically, the invention may support anatomical structures such as the vasculature in which many branches, intersections, bifurcations etc. can be found.

The present invention may be seen as reflecting this based on the connectivity which reflects connection paths (i.e. the blood flow along the vasculature).

The invention is not necessarily limited to vascular applications. It may be applicable to nervous and e.g. lymphatic or muscular networks and basically to all anatomical regions which have a fibrous or tubular structure.

In an embodiment, the method comprises a preceding step of obtaining reconstructed raw data of the at least one region, the raw data reflective of acquisition by means of a medical imaging system, such as computed tomography (CT) optionally using photon counting detectors or magnetic resonance imaging (MRI). For example, computed tomography medical imaging of an anatomical region involves an acquisition of raw data in the form of projection sinogram(s) that is (are) processed to reconstruct a medical 3D image of an anatomical region during a clinical diagnosis of the anatomical region as known in the art of the present disclosure. By further example, magnetic resonance imaging (MRI) of an anatomical region involves an acquisition of raw data in k-space that is processed to reconstruct a medical image of an anatomical region as known in the art of the present disclosure.

Direct volume rendering (DVR) may refer to rendering an image volume without prior segmentation of shapes of interest and in particular without mesh representation of surfaces of interest, e.g. using instead a ray tracing technique with certain opacity transfer functions.

A method of an embodiment of the invention may concern DVR. In contrast, "indirect" or surface based rendering may mean to first segment the structures of interest out of the overall image volume, then to convert them into a surface representation (e.g. triangle-meshes), and then to use a surface rendering technique.

Visualization of the voxels of interest preferably is a selective visualization, e.g. only or mainly the voxels of interest. However, the voxels may be weighted for rendering such that voxels that are not of interest appear less intensive, so that the step of rendering is free from an absolute selection of only voxels of interest. In other words, all voxels may still be rendered however te voxels of interest are rendered differently, e.g. with a different intensity.

In an embodiment, several starting point voxels, i.e. a set and/or a group of starting point voxels, may be used.
In an embodiment, at least one voxel of the plurality of voxels is excluded from being a neighbored voxel or starting point voxel, such as voxels at a boundary of the volume of interest.
In an embodiment, at least one voxel of the plurality of voxels represents a negative starting point voxel, such as voxels at a boundary of the volume of interest.

In an embodiment, the (ascending) aorta may be taken as starting point anatomy, when the vascular structure of interest is the coronary system. The starting point anatomy and the vascular structure of interest may be anatomically jointed/connected, i.e. by blood vessels. The starting point anatomy may be identified automatically by means of a model and/or identified by a radiologist/physician.

In an embodiment, the neighbored voxel only include the immediate neighbored voxels, i.e. the voxels sharing a common surface/corner (excluding indirect neighbors). The same Euclid distance of voxels from a "central" voxel may be used as criterion and/or a threshold for a neighborhood distance.

In an embodiment, the step of volume rendering includes volume rendering, per voxel, of the value of the connectivity or of the value of the connectivity only in a predetermined range or based on a combination of the value of the connectivity and an intensity value of the voxel. E.g. for volume rendering, the voxels may be weighted by means of their respective connectivity value.

In an embodiment, the step of volume rendering comprises volume rendering, per voxel of the connectivity value or of the connectivity value only in a predetermined range or of a combined value based on a combination of the connectivity value and any other value such as an intensity value of the voxel.

In other words, instead of rendering the original value of the voxel, the determined connectivity value is rendered. In a further embodiment, only the voxels with a connectivity value in a predetermined range are rendered. Voxels that do not comply with the predetermined range (which are outside of the predetermined range) are not rendered. In an alternative, such voxels that are outside of the predetermined range are rendered differently, for example these voxels are rendered with their original value (e.g. an intensity value) in contrast to being rendered with their connectivity value.

In an embodiment, the alignment value per voxel, comprises determining the alignment value per voxel based on a nxn-direction tensor, optionally at least n=3. The nxn-direction tensor determined for that voxel is indicative of the direction alignment of that voxel with respect to neighbored voxel that lie in the neighborhood of that voxel.

In a further embodiment, the direction tensor for determination of the alignment value may be three-dimensional (i.e. n= 3, 3x3) reflective of the three spatial dimensions x, y, z. Further dimensions, such as time and/or spectral characteristics, may be included, leading to n = 4 or 5.

In a further embodiment, derivative information in the nxn direction tensor may be determined based on derivatives of first, second or even higher order. The direction tensor may be reflective of derivative information indicative of local directions, per voxel of the plurality of voxels. For example, a Hessian matrix may be used as direction tensor, characterizing the local image geometry based on second-order derivative information. The Hessian matrix per neighbored voxel may include a three gradient volume and six independent 2nd-derivative volumes. The direction tensor may reflect the curvature of the image intensity and may be used to characterize the local image geometry. The direction tensor may be seen as serving to artificially "read" directional information into the voxel.

In an alternative embodiment, the determining of the alignment value per voxel (in other words representative for a direction or directional value per voxel) is determined based on an Eigenvector of the nxn direction tensor, per voxel of the plurality of voxels.

In a further embodiment, the determining of the alignment value per voxel is determined based on defining a nxn alignment tensor per voxel that is defined on the basis of a direction value per voxel of the plurality of voxels and on an outer product with the nxn alignment tensor per neighbored voxel. For example the value of n is at least 3..

Further optionally, the items of the nxn alignment tensor are weighted, optionally by using a standard 3D Gaussian smoothing-filter. The alignment tensor may have the dimension n = 3, 4, 5 ... (as for the direction tensor).

The nxn alignment tensor may be a so-called structure tensor, describing the distribution of the gradient in a neighborhood around a "point". The nxn alignment tensor may be a symmetric matrix.

The nxn alignment tensor may be adopted as the Eigenvector of length n, of highest absolute Eigenvalue magnitude, multiplied by e.g. the square root of the corresponding Eigenvalue.

The items of the nxn alignment tensor may be weighted, i.e. the sum of the neighbors are weighted, optionally by using a standard 3D Gaussian smoothing-filter.

The alignment value is optionally a scalar value. The alignment value may be determined based on the nxn alignment tensor, e.g. as the largest Eigenvalue thereof. If the alignment value is e.g. vectorial, or otherwise multi-valued, it may require some additional condensation into a scalar value which may serve for prioritization, which may be required to construct the connection path (e.g. blood flow) to each voxel. The alignment value may be a local value, i.e. the local alignment value, as the alignment in the neighborhood, i.e. in proximity of the voxel, is considered. As such, the alignment value may reflect two qualities: whether the voxels of the local neighborhood have indeed a pronounced directedness, and whether these directions are aligned.

The connectivity value may be seen as a local measure of connectivity, i.e. a local connectivity, as it is determined locally relative to the neighbors. The connectivity value may be locally different for every voxel, and it may depend incrementally on each local neighborhood, but it is accumulated along the entire connection path to each voxel. As such, the connectivity value may depend on the chain of local neighborhoods along the connection path. Based on the connectivity value, an expanding set of voxels of interest may be identified by means of a connection path of appropriate alignment values. The connection path may be identified based on an expanding sets of potential connection paths. This may be calculated sequentially or semi-parallel.

Optionally, the connectivity value from the starting point voxel to neighbored voxels is determined based on prioritized region-growing taking the alignment value per neighbored voxel into account.

The invention is also directed to a data processing apparatus comprising means for carrying out the method of the invention.

The invention is also directed to a computer program comprising instructions which, when the program is executed by a computer, cause the computer to carry out the method of the method of the invention.

The invention is also directed to a computer-readable medium comprising instructions which, when executed by a computer, cause the computer to carry out the method of the invention.

### BRIEF DESCRIPTION OF THE DRAWINGS

Fig. 1a, Fig. 1b and Fig. 1c schematically show two neighbored vessels.
Fig. 2a and Fig. 2b show different volume renderings according to an embodiment of the invention.
Fig. 3a and Fig. 3b show different volume renderings according to an embodiment of the invention.
Fig. 4a and Fig. 4b show volume renderings according to an embodiment of the invention.
Fig. 5 shows a volume rendering according to an embodiment of the invention.
Fig. 6 shows schematically a method for volume rendering according to an embodiment of the invention.

### DETAILED DESCRIPTION OF EMBODIMENTS

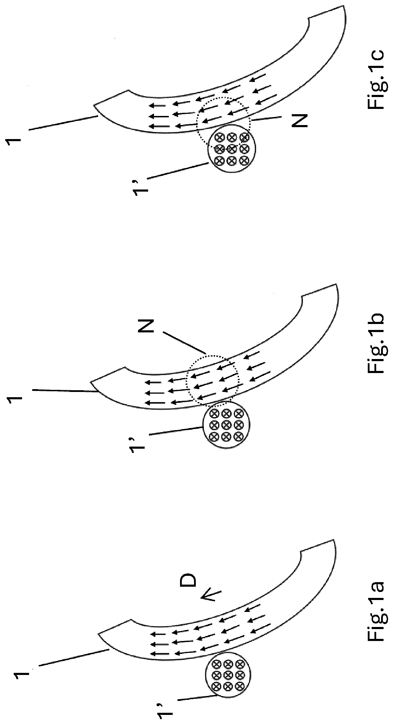

Fig. 1a, Fig. 1b and Fig. 1c schematically show two neighbored vessels 1, 1', wherein in Fig. 1b and 1c a neighborhood N is indicated. The principle of computation of local connectivity is indicated in Fig. 1 as follows:
Fig. 1a shows that two almost orthogonal vessel pieces 1, 1' touch/cross in 3D space. A first vessel 1 is shown elongated in the plane of the figure while a second vessel 1' is shown perpendicular to the plane of the figure. Local Hessian Eigenvectors are shown as arrows in the first vessel 1 (running in the plane, e.g. in the direction D) and in the second vessel 1' (running perpendicular to the plane) and indicate a weak low-level estimation of the direction vector per voxel.
Fig. 1b indicates a local structure tensor of a Gaussian-weighted neighborhood N, at a location yielding a high largest eigenvalue as the alignment value (high local vector alignment, as almost all arrows in N run in parallel).
Fig. 1c indicates a local structure tensor at a location/ neighborhood N yielding a low largest eigenvalue as the alignment value (low local vector alignment, as the arrows in the two vessels 1, 1' run perpendicular to each other, i.e. are less aligned relative to each other).

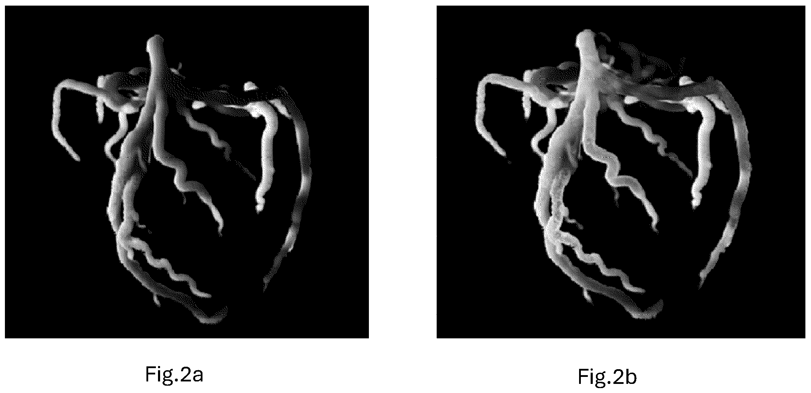

Fig. 2a and Fig. 2b show different volume renderings of a vascular region according to an embodiment of the invention. Fig. 2a and Fig. 2b represent examples of volume rendering of the aortic-connectivity values themselves.

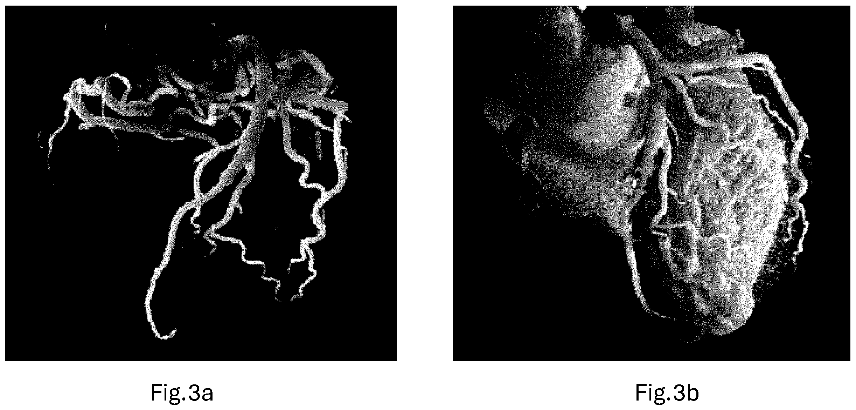

Fig. 3a and Fig. 3b show different volume renderings of a vascular region according to an embodiment of the invention. Fig. 3a and Fig. 3b show examples of volume rendering of the CT-densities (Hounsfield units) modulated (masked) by the voxel-wise aortic-connectivity, wherein Fig. 2(d) shows that lower thresholds (transfer-functions) also reveal the less-connected anatomies, here the left cardiac ventricle, conveying improved visual context.

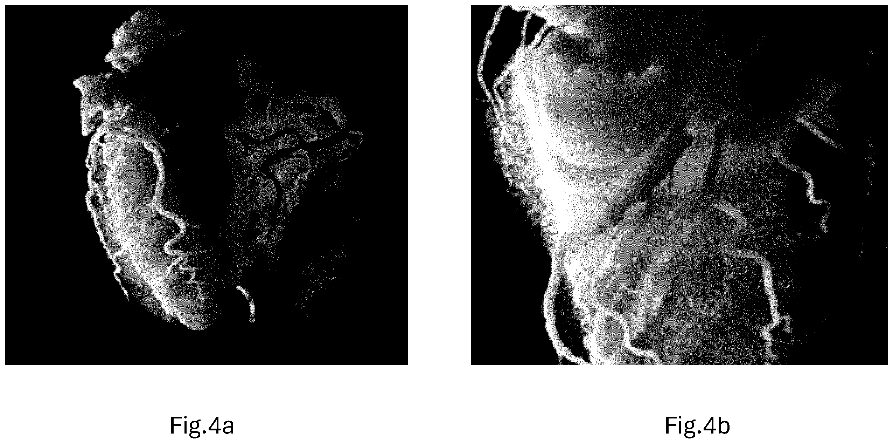

Fig. 4a and Fig. 4b show volume renderings based on the present invention and including an artificial coronary stent, which is depicted by the volume rendering. Segmentation problems may be less severe, since the directional connectivity is maintained through such a stent by design.

More specifically, in Fig. 4a, which in addition to the natural coronaries also shows a piece of artificial stenting, the intensities in the region are quite heterogeneous which may cause issues in prior art segmentation and rendering algorithms. By utilizing directional connectivity according to the present invention, this may be overcome, and results in showing the coronaries (including the artificial stent) more clearly as 'foreground', and the less connected ventricle as 'background'. For example, prior art renderings may be much more crowded and cluttered, while e.g. the cardiac wall (muscle layer) is transparent given the method of the invention, and may otherwise be opaque.

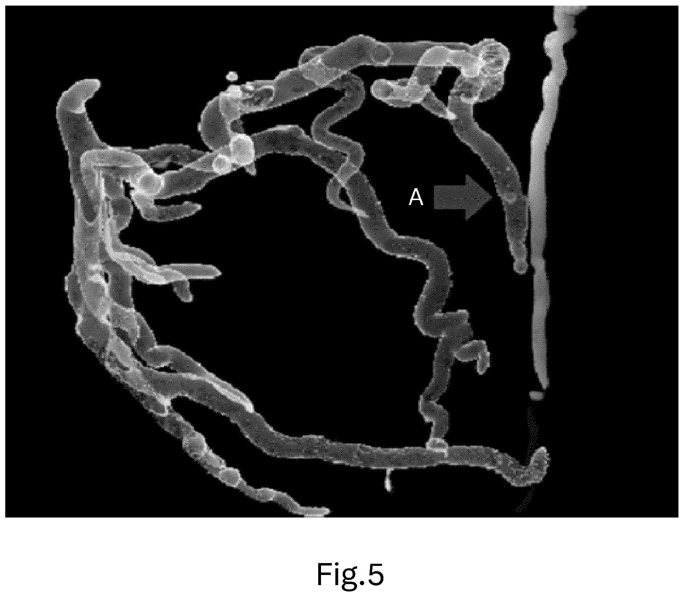

Fig. 5 shows a visualization of a volume rendering according an embodiment of the invention, including a non-coronary vessel (coming out of the lung and hugging the myocardium), which could possibly arise due to the touch point (see the arrow A in Fig. 5) with a true coronary. Such touch point may be identified interactively (e.g. by a mouse-click) by the user as an additional 'negative' seed, in order to be removed by a re-run of the minimum connection path computation. Hence, the volume rendering of Fig. 5 may be improved by using also additional negative starting points. With these, the non-coronary vessel may no longer be rendered, as it more strongly directionally connected to the 'negative' background.

The above described Fig. 2a, Fig 2b, Fig. 3a, Fig. 3b, Fig. 4a, Fig. 4b, Fig. 5a and Fig. 5b disclose renderings (visualizations) in black and white (greyscales). However, it is possible and foreseen that the rendering is performed in color (gradients).

In one embodiment of a method of the invention, one, more or all of the following steps may be performed to obtain e.g. a volume rendering shown in any of the previous described figures, i.e. Fig. 2a to 5:
1. Automatically detecting and delineating of the cardiac volume of interest, and the ascending aorta.
2. Optionally computing a reformat of the bounding box around the cardiac volume of interest by means of an isotropic resampling of the volume of interest, in order to save memory and to normalize spatial derivatives in x, y, z.
3. Weakly estimating the local direction by computing the local Hessian matrix for each voxel and efficiently storing the weakly estimated local direction as three gradient volumes and six independent 2nd-derivative volumes.
4. Adopting the local direction vector as the Eigenvector (unit length) of highest absolute Eigenvalue magnitude, multiplied by the square root of the corresponding Eigenvalue.
5. Estimating the local directional alignment using the local structure tensor, defined by the outer vector product of the local direction vector, yielding six independent components per voxel. All six components are locally accumulated in a weighted neighborhood by using a standard 3D Gaussian smoothing-filter applied to the tensor volume formed by all voxel-wise structure tensors. The scalar alignment value is then derived from the largest Eigenvalue.
5. Efficiently establishing the connectivity value from the seeds to each voxel using a prioritized region-growing (max-score-/min-cost-path propagation, implemented using e.g., a lazily sorted heap, which may be efficiently stopped when only negative heap elements remain).

Additionally or alternatively, in one embodiment, the method of the invention may comprise one, more or all of the following steps, to obtain e.g. a volume rendering shown in any of Fig. 2a to 5:
1. Selecting (manually or automatically) an anatomically unambiguous key component, which has e.g. low shape complexity and has low anatomical variation. As such, the ascending aorta (leaving the left cardiac ventricle) may automatically be segmented e.g., by model-based segmentation (MBS, analytical), or CNN-based semantic segmentation (direct, feature-free machine learning).
2. Propagating the connectivity (value) from (at least) one source of seed (starting point):
   the ascending aorta (as 'positive' seeds). Optionally, the boundary of the VOI ('negative' seeds, connecting to outside structures not of interest) may also represent a source of seed, i.e. a second source of seed. A prioritized region-growing may determine for each voxel the connectivity value (which may be seen as a minimum of maximizing path), where connection-paths are competing between 'positive' and `negative'-interest seeds. In the rendering, only voxels with 'positive' -interest path- connectivity (values) are considered.
3. Determining the local connectivity value by quantifying a scalar alignment value of local direction estimates of neighboring voxels (see Fig. 1a and Fig. 1b).
4. Rendering using a direct volume rendering (DVR):
   (a) the connectivity values of the connectivity-volume itself, or
   (b) a connectivity-masked image volume (which may be a 'conventional' full-energy image or a spectral derivative, e.g., a virtual mono-energy image), where voxels below a certain connectedness to positive seeds are ignored (discretely, binary), or
   (c) a connectivity-modulated image volume (conventional full-energy image or a spectral derivative, e.g., a virtual mono-energy image), with a function f(C)•g(I) , where f is a function of the local connectivity C(x), and g is a function of the local intensity I(x), which can be a 'conventional' full-energy image or a spectral result, e.g., a virtual mono-energy image.

In one embodiment of a method of the invention, one or more of the following additional steps/considerations may be applied, to obtain e.g. a volume rendering shown in any of Fig. 2a to 5:
1. Computing the voxel-wise vector computations in parallel using e.g. GPUs and/or Single Instruction, Multiple Data (SIMD)-CPUs.
2. Applying built-in hardware-accelerated types to tensor operations required for local vector storage and eigenvector computation, such as Float4 which is a vector type with four components allowing graphics operations.
3. For rendering, automatically deriving a virtual camera location, orientation and/or zoom (field of view) based on anatomical landmarks detection. Alternatively or additionally, a standard series of key images may be rendered automatically, for 2D printable reporting.
4. Determining the local connectivity value while taking into account, in addition to local directional information, the local connectivity value (propagation speed) depending on local radio density (Hounsfield units, or spectral properties), such that the assigned connectivity value is higher for density values typical for coronaries and lower for other anatomy-typical density ranges.
5. Interactively modifying an automatic selection by allowing a user, after the automatic computation of a vascular connectivity value and a graphical presentation of result(s) to the user, to interactively change a connectivity value threshold, to adjust the rendering to different levels of interest.
6. Interactively modifying an automatic selection by allowing a user to interactively identify an irrelevant non-coronary vessel (e.g., a pulmonary vessel touching a true coronary) as an additional 'negative' seed, in order to be removed by a re-run of a minimum connection path computation.
7. Interactively modifying an automatic selection by allowing a user to add one or more 'positive' on CABG origin locations, in order to be included by a re-run of the minimum connection path computation. This is advantageous because artificial vessels from Coronary Artery Bypass Graft Surgery (CABG) may possibly arise from locations far from the ascending aorta. Allowing to add 'positive' seeds helps in taking these artificial vessels into account.
8. Automatically adding of 'negative' seeds. At the most distal tree locations, coronary veins may be connecting with coronary arteries, and thus be included erroneously. An automatic shape model or other semantic segmentation may detect and segment the main cardiac veins (e.g., cardiac atria, right ventricle), and automatically generate 'negative' seeds on their segmentation surfaces, to further improve connectivity results against `spill-overs' into the coronary arteries.

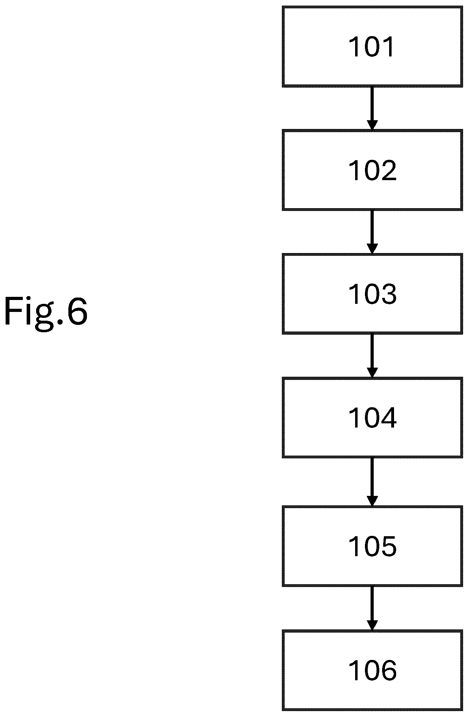

Fig. 6 shows schematically a method for volume rendering according to an embodiment of the invention. An embodiment of the method is disclosed in the form of a flowchart, in other words a block diagram.

The method comprises the step of receiving 101 data representing a plurality of voxels of the volume of interest representative of the at least one region.

The method comprises the step of obtaining 102, in the volume of interest, at least one starting point voxel representative of a starting point anatomy which is connected to the at least part of a structure.

As described above, such a starting point voxel may be a 'positive' seed. For example, the 'positive' seed or starting point voxel may be obtained from the ascending aorta, where the ascending aorta is the starting point anatomy.

The method comprises the step of determining 103, per voxel of the plurality of voxels of the volume of interest, an alignment value indicative of a direction D alignment of the respective voxel relative to neighbored voxels in a neighborhood N of the respective voxel.

There are multiple possibilities to determine the alignment value as disclosed above. In one example, the alignment value is a scalar value and the alignment value is determined based on a nxn alignment tensor, e.g. as the largest Eigenvalue thereof. If the alignment value is e.g. vectorial, or otherwise multi-valued, it may require some additional condensation into a scalar value which may serve for prioritization, which may be required to construct a connection path to each voxel. The alignment value may be a local value, i.e. the local alignment value, as the alignment in the neighborhood N, i.e. in proximity of the voxel, is considered. As such, the alignment value may reflect two qualities: whether the voxels of the local neighborhood have indeed a pronounced directedness, and whether these directions are aligned.

The method comprises the step of, based on the alignment value per voxel, determining 104 a connectivity value per voxel of the plurality of voxels relative to the starting point voxel, the connectivity value being indicative of an anatomical connection relative to the starting point anatomy.

The connectivity value may be seen as a local measure of connectivity, i.e. a local connectivity, as it is determined locally relative to the neighbors. The connectivity value may be locally different for every voxel, and it may depend incrementally on each local neighborhood, but it is accumulated along the entire connection path to each voxel. As such, the connectivity value may depend on the chain of local neighborhoods along the connection path. The connection path may be identified based on an expanding sets of potential connection paths. This may be calculated sequentially or semi-parallel.

Optionally, the connectivity value from the starting point voxel to neighbored voxels is determined based on prioritized region-growing taking the alignment value per neighbored voxel into account.

The method comprises the step of, based on the connectivity value, identifying 105 voxels of interest representing connected structures in the at least one region comprising the at least part of a structure.

Based on the connectivity value, an expanding set of voxels of interest may be identified by means of a connection path of appropriate alignment values. The connection path may be identified based on an expanding sets of potential connection paths. This may be calculated sequentially or semi-parallel.

The method comprises the step of volume rendering 106 of at least the voxels of interest for visualizing the at least part of a structure.

Visualization of the voxels of interest preferably is a selective visualization, e.g. only or mainly the voxels of interest. However, the voxels may be weighted for rendering such that voxels that are not of interest appear less intensive, so that the step of rendering is free from an absolute selection of only voxels of interest. In other words, all voxels may still be rendered however te voxels of interest are rendered differently, e.g. with a different intensity.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet, cloud or other wired or wireless telecommunication systems. If the term "for", "to", "adapted to" is used in the claims or description, it is noted the term "adapted to" is intended to be equivalent to the term "configured to". Any reference signs in the claims should not be construed as limiting the scope.

The flowchart and block diagram in Fig. 6 s illustrate the architecture, functionality, and operation of possible implementations of systems, methods, and computer program products according to various embodiments of the present invention. In this regard, each block in the flowchart or block diagram may represent a module, segment, or portion of instructions, which comprises one or more executable instructions for implementing the specified logical function(s).

In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved.

### LIST OF REFERENCE SIGNS:

- 1,1': vessel
- 101: receiving data
- 102: obtaining at least one starting point voxel
- 103: determining an alignment value
- 104: determining a connectivity value
- 105: identifying voxels of interest
- 106: volume rendering
- N: Neighborhood
- D: Direction
- A: touch point

## Claims

1. A computer-implemented method for volume rendering of at least one region comprising at least part of a structure (1, 1') in a volume of interest, the method comprising:
receiving (101) data representing a plurality of voxels of the volume of interest representative of the at least one region;
obtaining (102), in the volume of interest, at least one starting point voxel representative of a starting point anatomy which is connected to the at least part of a structure;
determining (103), per voxel of the plurality of voxels of the volume of interest, an alignment value indicative of a direction (D) alignment of the respective voxel relative to neighbored voxels in a neighborhood (N) of the respective voxel;
based on the alignment value per voxel, determining (104) a connectivity value per voxel of the plurality of voxels relative to the starting point voxel, the connectivity value being indicative of an anatomical connection relative to the starting point anatomy;
based on the connectivity value, identifying (105) voxels of interest representing connected structures in the at least one region comprising the at least part of a structure; and
volume rendering (106) of at least the voxels of interest for visualizing the at least part of a structure.

2. Method of claim 1, wherein the step of volume rendering comprises volume rendering, per voxel,
of the connectivity value; or
of the connectivity value only in a predetermined range; or
of a combined value based on a combination of the connectivity value and any other value such as an intensity value of the voxel.

3. Method of claim 1 or 2, wherein the step of determining the alignment value per voxel, comprises determining the alignment value per voxel based on a nxn-direction tensor, optionally at least n=3.

4. Method of claim 3, wherein the determining of the alignment value per voxel comprises the step of determining derivative information in the nxn-direction tensor that is based on derivatives of first or second order.

5. Method of claim 3 or 4, wherein the determining of the alignment value per voxel is determined based on an Eigenvector of the nxn-alignment tensor, per voxel of the plurality of voxels.

6. Method of claim 5, wherein an nxn alignment tensor per voxel is defined on the basis of a direction value per voxel of the plurality of voxels and on an outer product with the nxn alignment tensor per neighbored voxel, optionally at least n=3.

7. Method of claim 6, wherein the items of the nxn alignment tensor are weighted, optionally by using a standard 3D Gaussian smoothing-filter.

8. Method of claim 7, wherein the alignment value is determined based on the largest Eigenvalue of the nxn alignment tensor.

9. Method of any of the preceding claims, wherein the connectivity value from the starting point voxel to neighbored voxels is determined based on prioritized region-growing taking the alignment value per neighbored voxel into account.

10. Method of any of the preceding claims, wherein at least one voxel of the plurality of voxels is excluded from being a neighbored voxel or starting point voxel, such as voxels at a boundary of the volume of interest.

11. Method of any of the preceding claims, wherein at least one voxel of the plurality of voxels represents a negative starting point voxel, such as voxels at a boundary of the volume of interest.

12. Method of any of the preceding claims, wherein the method includes a preceding step of obtaining reconstructed raw data of the at least one vasculature region, the raw data reflective of acquisition by means of a medical imaging system, such as computed tomography, optionally using photon counting detectors.

13. A data processing apparatus comprising means for carrying out the method of any of the preceding claims.

14. A computer program comprising instructions which, when the program is executed by a computer, cause the computer to carry out the method of any of the preceding claims 1 to 12.

15. A computer-readable medium comprising instructions which, when executed by a computer, cause the computer to carry out the method of any of the preceding claims 1 to 12.
